# EUROPÄISCHE PATENTSCHRIFT

(11) **EP 2 603 485 B1**
(45) Veröffentlichungstag und Bekanntmachung des Hinweises auf die Patenterteilung: **14.01.2015**
(21) Anmeldenummer: 11748573.0
(22) Anmeldetag: 06.08.2011
(51) Int. Cl.: C07C 67/14, C07C 69/24

(54) **VERFAHREN ZUR HERSTELLUNG VON ACYLOXYBENZOESÄUREN**
METHOD FOR PRODUCING ACYLOXY BENZOIC ACIDS
PROCÉDÉ DE PRÉPARATION D'ACIDES ACYLOXYBENZOÏQUES

(30) Priorität: 13.08.2010 DE 102010034243
(43) Veröffentlichungstag der Anmeldung: 19.06.2013
(73) Patentinhaber: WeylChem Switzerland AG, 4132 Muttenz (CH)
(72) Erfinder: SAJITZ, Melanie, 58840 Plettenberg (DE); REINHARDT, Gerd, 65779 Kelkheim (DE); SCHEFFER, Isabel, 65929 Frankfurt (DE); JANITSCHEK, Werner, 65558 Heistenbach (DE); HERRGEN, Ina, 65428 Rüsselsheim (DE)
(74) Vertreter: Ackermann, Joachim
(86) Internationale Anmeldenummer: PCT/EP2011/003957
(87) Internationale Veröffentlichungsnummer: WO 2012/019743

(56) Entgegenhaltungen:
- WO-A2-2004/002979
- US-A- 5 891 838

## Beschreibung

Die vorliegende Erfindung betrifft ein Verfahren zur Herstellung von Acyloxybenzoesäuren ausgehend von para-Hydroxybenzoesäure und Carbonsäurehalogeniden.

Anorganische Persauerstoffverbindungen, insbesondere Wasserstoffperoxid und feste Persauerstoffverbindungen, die sich in Wasser unter Freisetzung von Wasserstoffperoxid lösen, wie Natriumperborat und Natriumpercarbonat, werden seit langem als Oxidationsmittel zu Desinfektions- und Bleichzwecken verwendet. Die Oxidationswirkung dieser Substanzen hängt in verdünnten Lösungen stark von der Temperatur ab. So erzielt man beispielsweise mit H₂O₂ oder Perborat in alkalischen Bleichflotten erst bei Temperaturen oberhalb von etwa 80 °C eine ausreichend schnelle Bleiche verschmutzter Textilien.

Bei niedrigeren Temperaturen kann die Oxidationswirkung der anorganischen Persauerstoffverbindungen durch Zusatz so genannter Bleichaktivatoren verbessert werden. Hierfür wurden in der Vergangenheit zahlreiche Vorschläge erarbeitet, vor allem aus den Stoffklassen der N- oder O-Acylverbindungen.

In den letzten Jahren waren die Verbindungen gemäß der Formel (la) 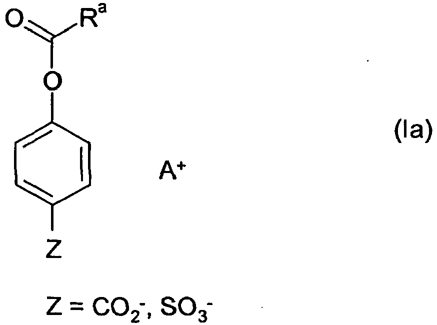 worin der Rest R^{a} insbesondere für eine lineare oder verzweigte, gesättigte Alkylgruppe mit 6 bis 22 Kohlenstoffatomen oder für eine lineare oder verzweigte, ein- oder mehrfach ungesättigte Alkenylgruppe mit 6 bis 22 Kohlenstoffatomen steht und
A⁺ insbesondere ein Alkali- oder Erdalkalimetallion und vorzugsweise ein Natriumion bedeutet, von Interesse als Aktivatoren für anorganische Peroxyverbindungen. Insbesondere war die Verwendung dieser Verbindungen als Bleichmittel bzw. als Peroxysäure-Vorstufe von Interesse.

Diese Peroxysäure-Vorstufen reagieren in wässriger Lösung mit den anorganischen Peroxyverbindungen wie Natriumpercarbonat oder Natriumperborat, die im Waschmittel enthalten sind, und bilden organische Peroxysäuren, die bei niedrigen Temperaturen (< 70 °C) viel effektiver bleichen als die anorganischen Peroxyverbindungen.

In besonderer Weise zeichnen sich Acyloxybenzoesäuren gemäß der Formel (Ib) 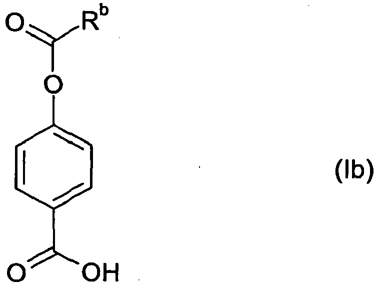 worin R^{b} insbesondere eine lineare oder verzweigte, gesättigte Alkylgruppe mit 6 bis 30 Kohlenstoffatomen, eine lineare oder verzweigte, ein- oder mehrfach ungesättigte Alkenylgruppe mit 6 bis 30 Kohlenstoffatomen, oder eine Arylgruppe mit 6 bis 30 Kohlenstoffatomen ist, sowie auch deren Salze, bei denen es sich insbesondere um Alkali- oder Erdalkalimetallsalze handelt aus, da sie nicht nur sehr gute Bleichaktivatoren sind, sondern sich vorteilhafter Weise nicht als hautsensibilisierend erweisen.

Sowohl die Phenolestersulfonate als auch die Acyloxybenzoesäuren und deren Salze sind prinzipiell z. B. durch Umsetzung von Carbonsäurechloriden wie beispielsweise Alkylsäurechlorid mit Phenolsulfonat oder para-Hydroxybenzoesäure nach der Schotten-Baumann-Reaktion zugänglich. Beispiele hierfür finden sich u. a. in EP 0 294 073, EP 0 164 786 oder WO 92/15556.

Die Acyloxybenzoesäuren können aus Säurechloriden und para-Hydroxybenzoesäure auch bei hohen Temperaturen > 30 °C hergestellt werden (JP 4194688 und WO 2004/002979). Ein Nachteil des Verfahrens ist jedoch die Bildung von Nebenkomponenten wie Dimere und Trimere der para-Hydroxybenzoesäure bzw. Estern der dimeren para-Hydroxybenzoesäure. Das Vorkommen derartiger Nebenkomponenten, die sich nicht abtrennen lassen, ist in Waschmitteln äußerst unerwünscht.

In US 5,891,838 wird ein Verfahren zur Herstellung von para-Decanoyloxybenzoesäure offenbart, das als Lösungsmittelgemisch Diethylether und Wasser vorsieht. Die Ausbeute von 40 % ist allerdings unbefriedigend und die großtechnische Handhabung von Diethylether aus Arbeitsschutzgründen unerwünscht.

Aufgabe der vorliegenden Erfindung war es, ein Verfahren zur Herstellung von langkettigen oder aromatischen Acyloxybenzoesäuren zur Verfügung zu stellen, das großtechnisch durchführbar ist, in hohen Ausbeuten zu den Acyloxybenzoesäuren führt, und wobei die Acyloxybenzoesäuren hinsichtlich Zusammensetzung und Qualität für den Einsatz in Wasch- und Reinigungsmitteln geeignet sind.

Es wurde nun gefunden, dass diese Aufgabe gelöst wird durch ein Verfahren zur Herstellung von Acyloxybenzoesäuren der Formel (I) 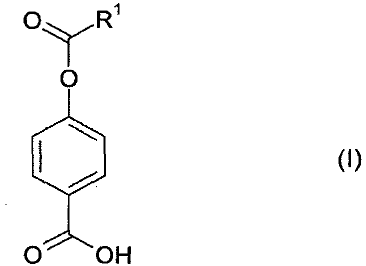 worin
- R¹: eine lineare oder verzweigte, gesättigte Alkylgruppe mit 6 bis 30 Kohlenstoffatomen, eine lineare oder verzweigte, ein- oder mehrfach ungesättigte Alkenylgruppe mit 6 bis 30 Kohlenstoffatomen, oder eine Arylgruppe mit 6 bis 30 Kohlenstoffatomen ist,
welches dadurch gekennzeichnet ist, dass
a) para-Hydroxybenzoesäure in einem Lösungsmittelgemisch enthaltend Wasser und ein organisches Lösungsmittel, wobei das eingesetzte Lösungsmittel iso-Propanol ist, mit einem Alkalihydroxid in einem molaren Verhältnis von Alkalihydroxid : para-Hydroxybenzoesäure ≥ 1,9 : 1 versetzt wird und erst danach ein Carbonsäurehalogenid der Formel R¹COHal, worin R¹ die oben angegebene Bedeutung besitzt und Hal ein Halogenid ist, bei einer Temperatur ≤ 25 °C zugegeben und zur Umsetzung gebracht wird,
b) das Reaktionsgemisch nach Schritt a) bei einer Temperatur ≤ 25 °C dann auf pH 6 bis 8,5 durch Zugabe von Säure eingestellt wird, wenn der pH-Wert am Ende von Schritt a) nicht bereits in diesem Bereich liegt,
c) das Reaktionsgemisch nach Schritt b), sofern dieser stattfindet und ansonsten nach Schritt a) auf eine Temperatur von 35 bis 80 °C erwärmt und danach durch Zugabe von Säure auf pH 1 bis 4 eingestellt wird, und
e) die gesamte Menge an Alkalihydroxid in Schritt a) vor der Zugabe des Carbonsäurehalogenids in den Reaktionsansatz gegeben und ansonsten in dem Verfahren keine weitere Base zugegeben wird.

Gegenstand der Erfindung ist daher ein Verfahren zur Herstellung von Acyloxybenzoesäuren der Formel (I) 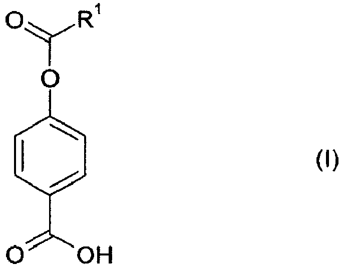 worin
- R¹: eine lineare oder verzweigte, gesättigte Alkylgruppe mit 6 bis 30, vorzugsweise 7 bis 15 und besonders bevorzugt 7 bis 11 Kohlenstoffatomen, eine lineare oder verzweigte, ein- oder mehrfach ungesättigte Alkenylgruppe mit 6 bis 30, vorzugsweise 7 bis 15 und besonders bevorzugt 7 bis 11 Kohlenstoffatomen, oder eine Arylgruppe mit 6 bis 30 Kohlenstoffatomen ist,
dadurch gekennzeichnet, dass
a) para-Hydroxybenzoesäure in einem Lösungsmittelgemisch enthaltend Wasser und ein organisches Lösungsmittel, wobei das eingesetzte Lösungsmittel iso-Propanol ist, mit einem Alkalihydroxid in einem molaren Verhältnis von Alkalihydroxid : para-Hydroxybenzoesäure ≥ 1,9 : 1 versetzt wird und erst danach ein Carbonsäurehalogenid der Formel R¹COHal, worin R¹ die oben angegebene Bedeutung besitzt und Hal ein Halogenid ist, bei einer Temperatur ≤ 25 °C, vorzugsweise bei 0 bis 25 °C und besonders bevorzugt bei 0 bis 15 °C, zugegeben und zur Umsetzung gebracht wird,
b) das Reaktionsgemisch nach Schritt a) bei einer Temperatur ≤ 25 °C, vorzugsweise bei 0 bis 25 °C und besonders bevorzugt bei 0 bis 15 °C, dann auf pH 6 bis 8,5 und vorzugsweise 6 bis 8 durch Zugabe von Säure eingestellt wird, wenn der pH-Wert am Ende von Schritt a) nicht bereits in diesem Bereich liegt,
c) das Reaktionsgemisch nach Schritt b), sofern dieser stattfindet und ansonsten nach Schritt a) auf eine Temperatur von 35 bis 80 °C, vorzugsweise von 50 bis 70 °C, erwärmt und danach durch Zugabe von Säure, vorzugsweise HCl, auf pH 1 bis 4 eingestellt wird, und
e) die gesamte Menge an Alkalihydroxid in Schritt a) vor der Zugabe des Carbonsäurehalogenids in den Reaktionsansatz gegeben und ansonsten in dem Verfahren keine weitere Base zugegeben wird.

In Schritt a) des erfindungsgemäßen Verfahrens findet sowohl die Zugabe als auch die Umsetzung des Carbonsäurehalogenids bei einer Temperatur ≤ 25 °C, vorzugsweise bei 0 bis 25 °C und besonders bevorzugt bei 0 bis 15 °C, statt.

Das erfindungsgemäße Verfahren besitzt den Vorteil, dass die Acyloxybenzoesäuren der Formel (I) in einfacher Weise aus dem Reaktionsgemisch abfiltriert werden können. Die nach dem erfindungsgemäßen Verfahren erhaltenen Acyloxybenzoesäuren sind weiß bis cremefarben und können in Form von Pulver oder Granulat zur Herstellung von Wasch- und Reinigungsmitteln eingesetzt werden. Besonders vorteilhaft ist, dass die nach dem erfindungsgemäßen Verfahren erhaltenen Acyloxybenzoesäuren eine sehr hohe Reinheit aufweisen und Nebenkomponenten wie z. B. Dimere oder Trimere der para-Hydroxybenzoesäure vorzugsweise in einer Menge < 1,0 Gew.-% enthalten. Im Gegensatz zu Acyloxybenzoesäuren aus anderen Verfahren, die in der Regel Nebenprodukte enthalten, sind die Acyloxybenzoesäuren aus dem erfindungsgemäßen Verfahren aufgrund ihrer hohen Reinheit besonders gut geeignet, in Wasch- und Reinigungsmitteln eingesetzt zu werden.

Des Weiteren besitzt das erfindungsgemäße Verfahren den Vorteil, dass eine starke Niederschlagsbildung bei der Zugabe des Carbonsäurehalogenids in Schritt a) des erfindungsgemäßen Verfahrens unterbleibt.

In Schritt a) des erfindungsgemäßen Verfahrens wird die gesamte Menge an Alkalihydroxid vor der Zugabe des Carbonsäurehalogenids in den Reaktionsansatz gegeben und ansonsten in dem Verfahren keine weitere Base zugegeben. Der Vorteil dieser Vorgehensweise liegt z.B. darin, dass sich eine pH-Wert-Kontrolle während der Umsetzung in Schritt a) erübrigt und sich das Verfahren insofern in einfacher Weise durchführen lässt.

Sofern sich der pH-Wert während des Schrittes a) soweit erniedrigt, dass er am Ende von Schritt a) von 6 bis 8,5 und vorzugsweise von 6 bis 8 beträgt, entfällt Schritt b) des erfindungsgemäßen Verfahrens.

Bevorzugte Carbonsäurehalogenide der Formel R¹COHal sind diejenigen, worin Hal Cl oder Br ist. Besonders bevorzugte Carbonsäurehalogenide der Formel R¹COHal sind die Carbonsäurechloride gemäß der Formel (II)

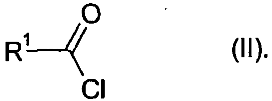

In dem Fall, dass der Rest R¹ eine Arylgruppe darstellt, ist er vorzugsweise eine Phenylgruppe oder eine Phenylgruppe, die mit 1 bis 3 Methylgruppen substituiert ist. Unter diesen substituierten Phenylgruppen ist die Gruppe -C₄H₆-CH₃ wiederum bevorzugt. Unter den Arylgruppen ist jedoch die (unsubstituierte) Phenylgruppe besonders bevorzugt.

Vorzugsweise ist der Rest R¹ jedoch eine Alkyl- oder Alkenylgruppe. Beispiele für den Carbonsäurehalogeniden R¹-COHal zugrunde liegende Carbonsäuren R¹-COOH sind Heptansäure, Octansäure, Methyloctansäure, Nonansäure, 3,3,5-Isononansäure, Decansäure, Undecansäure, Undecensäure, Dodecansäure, Tetradecansäure, gehärtete Talgfettsäure und Octadecansäure.

Besonders bevorzugt ist der Rest R¹ eine Alkylgruppe. Insbesondere bevorzugt sind die den Carbonsäurehalogeniden zugrunde liegenden Carbonsäuren R¹-COOH ausgewählt aus der Gruppe bestehend aus Octansäure, Nonansäure, 3,3,5-Isononansäure, Decansäure und Dodecansäure. Unter diesen sind wiederum Nonansäure und Decansäure bevorzugt.

Vorzugsweise wird das erfindungsgemäße Verfahren derart ausgeführt, dass nach Schritt c) das Reaktionsgemisch auf eine Temperatur < 35 °C, besonders bevorzugt < 30 °C, insbesondere bevorzugt von 0 bis 30 °C und außerordentlich bevorzugt von 5 bis 25 °C abgekühlt wird (Schritt d)). Hierdurch kann insbesondere eine Ausbeutesteigerung erzielt werden.

Das bei der Acylierungsreaktion in Schritt a) des erfindungsgemäßen Verfahrens zugesetzte Alkalihydroxid hat die Aufgabe, durch Bildung des Phenolats die Reaktion zu ermöglichen. Vorzugsweise ist das in Schritt a) eingesetzte Alkalihydroxid KOH oder NaOH und besonders bevorzugt ist es KOH.

Das in Schritt a) des erfindungsgemäßen Verfahrens eingesetzte organische Lösungsmittel ist iso-Propanol.

Vorzugsweise besitzt die in Schritt b) (sofern dieser Schritt b) stattfindet) und in Schritt c) des erfindungsgemäßen Verfahrens eingesetzte Säure einen pKa-Wert kleiner oder gleich 4,0. Besonders bevorzugt ist diese Säure H₂SO₄ oder HCl und insbesondere bevorzugt HCl.

Vorzugsweise ist das Gewichtsverhältnis von Wasser zu iso-Propanol in Schritt a) von 5 : 1 bis 1 : 5 und besonders bevorzugt von 3 : 1 bis 1 : 2.

Vorzugsweise ist das Gewichtsverhältnis von Wasser zu para-Hydroxybenzoesäure in Schritt a) von 2 : 1 bis 10 : 1 und besonders bevorzugt von 2 : 1 bis 6 : 1.

Vorzugsweise ist das molare Verhältnis des Carbonsäurehalogenids der Formel R¹COHal zu para-Hydroxybenzoesäure von 0,75 : 1 bis 1,5 : 1, besonders bevorzugt von 0,9 : 1 bis 1,1 : 1 und insbesondere bevorzugt 1 : 1.

Vorzugsweise ist das molare Verhältnis von Alkalihydroxid : para-Hydroxybenzoesäure von 1,9 : 1 bis 3 : 1, besonders bevorzugt von 2 : 1 bis 2,5 : 1 und insbesondere bevorzugt von 2 : 1 bis 2,2 : 1.

Zur Isolierung und Aufreinigung der nach dem erfindungsgemäßen Verfahren hergestellten Acyloxybenzoesäuren der Formel (I) wird vorzugsweise wie folgt vorgegangen: Das Reaktionsgemisch wird über konventionelle Trennmethoden filtriert (Filterapparate), vorzugsweise bei Raumtemperatur, und der Rückstand so lange mit Wasser gewaschen, bis kein Salz mehr vorhanden ist. Die Filtration findet vorzugsweise nach Schritt d) statt. Die gebildete Acyloxybenzoesäure fällt in hohen Ausbeuten in Form eines weißen Pulvers an, das durch konventionelle Methoden getrocknet werden kann.

Das Endprodukt enthält allenfalls Spuren der Carbonsäure R¹-COOH. Nicht umgesetzte para-Hydroxybenzoesäure und Salze wie beispielsweise Kaliumchlorid können durch Waschen mit Wasser vollständig aus dem Filterkuchen entfernt werden.

Das Produkt des erfindungsgemäßen Verfahrens kann in vorteilhafter Weise als Aktivator für Wasserstoffperoxid eingesetzt werden.

Die nach dem erfindungsgemäßen Verfahren erhältlichen Acyloxybenzoesäuren sind in ihrer Wirkung als Bleichaktivator signifikant effektiver als Acyloxybenzoesäuren, die nach herkömmlichen Verfahren hergestellt werden. Die Persäure-Freisetzung erfolgt bei den nach dem erfindungsgemäßen Verfahren erhältlichen Acyloxybenzoesäuren wesentlich früher als bei nach konventionellen Verfahren hergestellten Acyloxybenzoesäuren.

Die nach dem erfindungsgemäßen Verfahren erhältlichen Acyloxybenzoesäuren können als Persalzaktivatoren in flüssigen oder pulverförmigen Wasch- und Reinigungsmitteln wie pulverförmigen Vollwaschmitteln, Fleckensalzen oder pulverförmigen Maschinengeschirrspülmitteln eingesetzt werden. Zur Erhöhung der Lagerstabilität in diesen Formulierungen können sie, wie dem Fachmann bekannt ist, in eine granulare Form überführt werden. Durch die Aktivierung lässt sich beispielsweise in Wasch- und Reinigungsmitteln die Bleichleistung der anorganischen Peroxyverbindungen / Wasserstoffperoxid verbessern oder in Desinfektionsmitteln die Desinfektionsleistung steigern.

Die folgenden Beispiele sollen die Erfindung näher erläutern ohne sie jedoch darauf einzuschränken.

### Beispiele

### Beispiel 1: Synthese von para-Decanoyloxy-benzoesäure (DOBA)

138,1 g (1,0 mol) para-Hydroxybenzoesäure wurden zunächst in 400 ml Wasser und 600 ml Isopropanol gelöst und bei 0 bis 5 °C mit 235,7 g KOH-Lösung (50 Gew.-%ige wässrige Lösung, 2,1 mol) versetzt. Der resultierende pH-Wert war 14. Zu dieser Lösung wurden dann innerhalb von drei Stunden bei 0 bis 5 °C 190,7 g (1,0 mol) Decansäurechlorid zudosiert und 30 Minuten bei 0 bis 5 °C nachgerührt. Am Ende des Nachrührens betrug der pH-Wert des Reaktionsansatzes 9,7. Anschließend wurde das Reaktionsgemisch bei 0 bis 5 °C mit 25,6 g HCl-Lösung (32 Gew.-%ige wässrige Lösung) auf pH 8 gestellt, die komplette Lösung auf 55 bis 60 °C erhitzt und dann mit 100 g HCl-Lösung (32 Gew.-%ige wässrige Lösung) auf pH 1,5 bis 3 gestellt. Das Reaktionsgemisch wurde auf 20 bis 25 °C gekühlt, der Feststoff über eine Saugnutsche abfiltriert und zehnfach mit 150 ml Wasser gewaschen. Nach dem Trocknen im Vakuum bei 100 °C betrug die Ausbeute 268,3 g (91,8 % der Theorie). Das Produkt war laut HPLC- und NMR-Messung frei von Decansäure und nicht umgesetzter para-Hydroxybenzoesäure. Die Reinheit des Produkts ist > 99,9 Gew.-%.

### Beispiel 2: Synthese von para-Decanoyloxy-benzoesäure (DOBA)

138,1 g (1,0 mol) para-Hydroxybenzoesäure wurden zunächst in 400 ml Wasser und 600 ml Isopropanol gelöst und bei 20 bis 25 °C mit 235,7 g KOH-Lösung (50 Gew.-%ige wässrige Lösung, 2,1 mol) versetzt. Der resultierende pH-Wert war 14. Zu dieser Lösung wurden dann innerhalb von drei Stunden bei 20 bis 25 °C 190,7 g (1,0 mol) Decansäurechlorid zudosiert und 30 Minuten bei 20 bis 25 °C nachgerührt. Anschließend wurde die komplette Lösung auf 55 bis 60 °C erhitzt und dann mit 124,7 g HCl-Lösung (32 Gew.-%ige wässrige Lösung) auf pH 1,5 bis 3 gestellt. Das Reaktionsgemisch wurde auf 20 bis 25 °C gekühlt, der Feststoff über eine Saugnutsche abfiltriert und zehnfach mit 150 ml Wasser gewaschen. Nach dem Trocknen im Vakuum bei 100 °C betrug die Ausbeute 237,5 g (81,2 % der Theorie). Das Produkt war laut HPLC- und NMR-Messung frei von Decansäure und nicht umgesetzter para-Hydroxybenzoesäure. Die Reinheit des Produkts ist > 99,9 Gew.-%.

### Beispiel 3: Synthese von para-Decanoyloxy-benzoesäure (DOBA)

138,1 g (1,0 mol) para-Hydroxybenzoesäure wurden zunächst in 400 ml Wasser und 600 ml Isopropanol gelöst und bei 10 bis 15 °C mit 235,7 g KOH-Lösung (50 Gew.-%ige wässrige Lösung, 2,1 mol) versetzt. Der resultierende pH-Wert war 14. Zu dieser Lösung wurden dann innerhalb von drei Stunden bei 10 bis 15 °C 190,7 g (1,0 mol) Decansäurechlorid zudosiert und 30 Minuten bei 10 bis 15 °C nachgerührt. Am Ende des Nachrührens betrug der pH-Wert des Reaktionsansatzes 8,7. Anschließend wurde das Reaktionsgemisch bei 10 bis 15 °C mit 10,4 g HCl-Lösung (32 Gew.-%ige wässrige Lösung) auf pH 8 gestellt, die komplette Lösung auf 55 bis 60 °C erhitzt und dann mit 115 g HCl-Lösung (32 Gew.-%ige wässrige Lösung) auf pH 1,5 bis 3 gestellt. Das Reaktionsgemisch wurde auf 20 bis 25 °C gekühlt, der Feststoff über eine Saugnutsche abfiltriert und zehnfach mit 150 ml Wasser gewaschen. Nach dem Trocknen im Vakuum bei 100 °C betrug die Ausbeute 257,0 g (87,9 % der Theorie). Das Produkt war laut HPLC- und NMR-Messung frei von Decansäure und nicht umgesetzter para-Hydroxybenzoesäure. Die Reinheit des Produkts ist > 99,9 Gew.-%.

### Beispiel 4: Synthese von para-Decanoyloxy-benzoesäure (DOBA)

69,1 g (0,5 mol) para-Hydroxybenzoesäure wurden zunächst in 200 ml Wasser und 300 ml Isopropanol gelöst und bei 10 bis 15 °C mit 127,5 g NaOH-Lösung (32 Gew.-%ige wässrige Lösung, 1,02 mol) versetzt. Der resultierende pH-Wert war 13,7. Zu dieser Lösung wurden dann innerhalb von drei Stunden bei 10 bis 55 °C 95,4 g (0,5 mol) Decansäurechlorid zudosiert und 30 Minuten bei 10 bis 15 °C nachgerührt. Anschließend wurde die komplette Lösung auf 55 bis 60 °C erhitzt und dann mit 60,0 g HCl-Lösung (32 Gew.-%ige wässrige Lösung) auf pH 1,5 bis 3 gestellt. Das Reaktionsgemisch wurde auf 20 bis 25 °C gekühlt, der Feststoff über eine Saugnutsche abfiltriert und zehnfach mit 75 ml Wasser gewaschen. Nach dem Trocknen im Vakuum bei 100 °C betrug die Ausbeute 124,8 g (85,4 % der Theorie). Das Produkt war laut HPLC- und NMR-Messung frei von Decansäure und nicht umgesetzter para-Hydroxybenzoesäure. Die Reinheit des Produkts ist > 99,9 Gew.-%.

### Vergleichsbeispiel 1: Synthese von para-Decanoyloxy-benzoesäure (DOBA)

116,3 g (0,61 mol) Decansäurechlorid wurden in 300 ml Xylol auf 125 °C geheizt und innerhalb von 6 Stunden portionsweise 69,1 g (0,5 mol) 4-Hydroxybenzoesäure eingetragen. Der Ansatz wurde 1 Stunde bei 125 °C nachgerührt, auf Raumtemperatur abgekühlt, abgesaugt und dreimal mit 45 ml Xylol gewaschen. Nach dem Trocknen im Vakuum bei 100 °C betrug die Ausbeute 108,4 g (74 % der Theorie). Das Produkt war laut HPLC- und NMR-Messung frei von Decansäure und nicht umgesetzter para-Hydroxybenzoesäure, aber enthielt 0,4 Gew.-% Nebenprodukte wie Dimere und Trimere der para-Hydroxybenzoesäure.

### Vergleichsbeispiel 2: Synthese von para-Decanoyloxy-benzoesäure (DOBA) nach US 5,891,838, Beispiel XV

DOBA wurde nach Beispiel XV der US 5,891,838 hergestellt. Nach der Filtration des Produkts (welches eine schmierige Konsistenz aufwies, wodurch wiederum die Filtration nachteiliger Weise sehr lange dauerte), wurde wie folgt aufgearbeitet: Der Rückstand der Filtration wurde mehrmals mit Wasser gewaschen und das Produkt bei 100 °C im Vakuum getrocknet. Die Ausbeute betrug 90,6 g. Das Produkt enthielt laut HPLC- und NMR-Messung 10,9 Gew.-% Decansäure, 30,4 Gew.-% DOBA und 41 Gew.-% para-Hydroxybenzoesäure. Anschließend wurde das Produkt wie in US 5,891,838 zur Reinigung umkristallisiert und für die Messung der Persäurekinetik herangezogen (siehe nachfolgendes Beispiel 5).

### Beispiel 5: Bestimmung der Persäurekinetik von para-Decanoyloxy-benzoesäure (DOBA) aus dem erfindungsgemäßen Beispiel 1 und den Vergleichsbeispielen 1 und 2

Die Persäurekinetik wurde mittels iodometrischer Titration mit Natriumthiosulfat-Lösung bestimmt.

Die Messung beruht darauf, dass para-Decanoyloxy-benzoesäure (DOBA) und Wasserstoffperoxid in wässriger Lösung zu Perdecansäure und para-Hydroxybenzoesäure reagieren (sofern anorganische Peroxide verwendet werden, reagieren diese in wässriger Lösung zu Wasserstoffperoxid). Die Reaktion zwischen DOBA und Wasserstoffperoxid erfolgt in verdünnter wässriger Lösung bei einem pH-Wert von 10 bis 11 bei 20 °C schnell und quantitativ. Die gebildete Perdecansäure kann dann neben dem im Überschuss vorhandenen Wasserstoffperoxid iodometrisch bestimmt werden. Die Perdecansäure ist wesentlich reaktiver als Wasserstoffperoxid und oxidiert in schwach saurem Medium und bei tiefer Temperatur mit zugegebenem Iodid I⁻ (z. B. zugegeben in der Form von Kaliumiodid) sofort zu Iod I₂. Das entstandene Iod kann dann mit Natriumthiosulfat titriert werden. Aus der gefundenen Menge an Iod kann dann die entsprechende Menge an Perdecansäure berechnet werden.

Im Einzelnen wurde wie folgt vorgegangen:
In einem 2 Liter-Becherglas wurden 1 Liter entsalztes Wasser von 20 °C vorgelegt und gerührt. Hierzu wurden 1,5 g Natriumpercarbonat und 8 g eines Standardwaschmittels ("IEC 60 456 Typ A*" der WFK Testgewebe GmbH) gegeben und 2 Minuten vorgelöst. Anschließend wurden 0,25 g des zu untersuchenden DOBA zugegeben. Nach 3 Minuten wurden 50 ml abpipettiert und in ein 250 ml Becherglas auf 50 g Eis aus entsalztem Wasser und 10 ml Essigsäure (20 Gew.-%ige wässrige Lösung) gegeben. Anschließend wurden 5 ml wässrige Kaliumiodid-Lösung (10 Gew.-%ige wässrige Lösung) zugegeben und mit Natriumthiosulfat-Lösung (0,01 molare wässrige Lösung) titriert.

Zur Titration wurden ein "Titrino DMS 716" oder "Basic 794" (Metrohm) mit einer 50er Wechseleinheit und Keyboard sowie ein "Ti Stand 727" (Metrohm) mit gezogener Bürettenspitze, Rührstab und kombinierter Platinelektrode verwendet.

Die nächsten Proben wurden nach bestimmten Zeiten nach der DOBA-Zugabe gezogen und wie oben beschrieben titriert. Die Menge an Perdecansäure nähert sich mit zunehmender Zeit der Probennahme einem Maximalwert an und bleibt dann bei noch später gezogenen Proben konstant. Dieser Maximalwert für die Menge an Perdecansäure wird auf 100 % gesetzt. Die Mengen an Perdecansäure für die anderen Proben werden dann in Relation zu diesen 100 % gesetzt.

Die Ergebnisse der Bestimmung der Persäurekinetik, d. h. die Ergebnisse der Bestimmung der Menge an Perdecansäure in Abhängigkeit von der Zeit, sind in Tabelle 1 gezeigt.

**Tabelle 1: Ergebnisse der Bestimmung der Persäurekinetik von para-Decanoyloxy-benzoesäure (DOBA)**

| Zeit [Min.] | Menge an Perdecansäure [%] | | |
|---|---|---|---|
| | Verwendung von DOBA aus erfindungsgemäßem Beispiel 1 | Verwendung von DOBA aus Vergleichsbeispiel 1 | Verwendung von DOBA aus Vergleichsbeispiel 2 |
| 3 | 90,6 | 66,85 | 82,04 |
| 6 | 94,9 | 82,92 | 84,28 |
| 9 | 99,1 | 87,98 | 88,00 |
| 12 | 100,00 | 92,91 | 93,38 |
| 15 | | 96,44 | 94,32 |
| 18 | | 100,00 | 100,00 |

Die Ergebnisse der Tabelle 1 beschreiben die Wirkstoff-Freisetzung aus para-Decanoyloxy-benzoesäure (DOBA), d. h. hier die Freisetzung von Perdecansäure, in Abhängigkeit von der Zeit. Sie lassen erkennen, dass der Wirkstoff aus dem DOBA, welches nach dem erfindungsgemäßen Beispiel 1 hergestellt worden ist, schneller freigesetzt wird, als der Wirkstoff aus den DOBAs, die nach den Vergleichsbeispielen 1 und 2 hergestellt worden sind. Es zeigt sich nämlich an den Ergebnissen aus Tabelle 1, dass das DOBA, welches nach den Vergleichsbeispielen 1 und 2 hergestellt worden ist, erst nach 18 Minuten 100 % der Perdecansäure generiert hat, wohingegen das DOBA, welches nach dem erfindungsgemäßen Beispiel 1 hergestellt worden ist, bereits nach 12 Minuten diesen Wert erreicht.

Das erfindungsgemäße Verfahren stellt somit im Hinblick auf den Einsatz der damit erhaltenen Acyloxybenzoesäuren der Formel (I) in Waschmitteln eine signifikante Verbesserung dar.

### Beispiel 6: Synthese von para-Decanoyloxy-benzoesäure (DOBA)

Das Verfahren entsprach Beispiel 1, jedoch wurde bei einer Ansatzgröße von 5 mol die Dosierzeit des Decansäurechlorides anstatt drei Stunden auf 20 Minuten verändert. Die Ausbeute beträgt 1242,62 kg (85%). Die Reinheit des Produkts ist > 99,9 Gew.-%.
Der d₁₀ Wert der Partikel ist 10,358 µm, der d₅₀ Wert ist 52,548 µm und der d₉₀ Wert ist 140,059 µm.

### Beispiel 7: Synthese von para-Benzoyloxy-benzoesäure (BOBA)

138,1 g (1,0 mol) para-Hydroxybenzoesäure wurden zunächst in 400 ml Wasser und 600 ml Isopropanol gelöst und bei 0 bis 5 °C mit 235,7 g KOH-Lösung (50 Gew.-%ige wässrige Lösung, 2,1 mol) versetzt. Der resultierende pH-Wert war 14. Zu dieser Lösung wurden dann innerhalb von einer Stunde bei 0 bis 5 °C 140,4 g (1,0 mol) Benzoylchlorid zudosiert und 30 Minuten bei 0 bis 5 °C nachgerührt. Am Ende des Nachrührens betrug der pH-Wert des Reaktionsansatzes 9,3. Anschließend wurde das Reaktionsgemisch bei 0 bis 5 °C mit 25,0 g HCl-Lösung (32 Gew.-%ige wässrige Lösung) auf pH 8 gestellt, die komplette Lösung auf 55 bis 60 °C erhitzt und dann mit 100 g HCl-Lösung (32 Gew.-%ige wässrige Lösung) auf pH 1,5 bis 3 gestellt. Das Reaktionsgemisch wurde auf 20 bis 25 °C gekühlt, der Feststoff über eine Saugnutsche abfiltriert und zehnfach mit 150 ml Wasser gewaschen. Nach dem Trocknen im Vakuum bei 100 °C betrug die Ausbeute 220,5 g (91,0 % der Theorie). Das Produkt war laut HPLC- und NMR-Messung frei von Benzoesäure und nicht umgesetzter para-Hydroxybenzoesäure. Die Reinheit des Produkts ist > 99,9 Gew.-%.

### Beispiel 8: Synthese von para-Nonanoyloxy-benzoesäure (NOBA)

138,1 g (1,0 mol) para-Hydroxybenzoesäure wurden zunächst in 500 ml Wasser und 500 ml Isopropanol gelöst und bei 0 bis 5 °C mit 230,0 g KOH-Lösung (50 Gew.-%ige wässrige Lösung, 2,05 mol) versetzt. Der resultierende pH-Wert war 13,5. Zu dieser Lösung wurden dann innerhalb von einer Stunde bei 0 bis 5 °C 176,6 g (1,0 mol) Nonansäurechlorid zudosiert und 30 Minuten bei 0 bis 5 °C nachgerührt. Am Ende des Nachrührens betrug der pH-Wert des Reaktionsansatzes 9,0. Anschließend wurde das Reaktionsgemisch bei 0 bis 5 °C mit 25,0 g HCl-Lösung (32 Gew.-%ige wässrige Lösung) auf pH 8 gestellt, die komplette Lösung auf 55 bis 60 °C erhitzt und dann mit 100 g HCl-Lösung (32 Gew.-%ige wässrige Lösung) auf pH 1,5 bis 3 gestellt. Das Reaktionsgemisch wurde auf 20 bis 25 °C gekühlt, der Feststoff über eine Saugnutsche abfiltriert und zehnfach mit 150 ml Wasser gewaschen. Nach dem Trocknen im Vakuum bei 100 °C betrug die Ausbeute 255,95 g (92,0 % der Theorie). Das Produkt war laut HPLC- und NMR-Messung frei von Nonansäure und nicht umgesetzter para-Hydroxybenzoesäure. Die Reinheit des Produkts ist > 99,9 Gew.-%.

### Beispiel 9: Synthese von para-Decanoyloxy-benzoesäure (DOBA)

172,7 g (1,25 mol) para-Hydroxybenzoesäure wurden zunächst in 400 ml Wasser und 600 ml Isopropanol gelöst und bei 10 bis 15 °C mit 287,3 g KOH-Lösung (50 Gew.-%ige wässrige Lösung, 2,56 mol) versetzt. Der resultierende pH-Wert war 14. Zu dieser Lösung wurden dann innerhalb von zwei Stunden bei 10 bis 15 °C 238,4 g (1,25 mol) Decansäurechlorid zudosiert und 15 Minuten bei 10 bis 15 °C nachgerührt. Am Ende des Nachrührens betrug der pH-Wert des Reaktionsansatzes 9,7. Anschließend wurde das Reaktionsgemisch bei 10 bis 15 °C mit 31,3 g HCl-Lösung (32 Gew.-%ige wässrige Lösung) versetzt, die komplette Lösung auf 55 bis 60 °C erhitzt und dann mit 125 g HCl-Lösung (32 Gew.-%ige wässrige Lösung) auf pH 1,5 bis 3 gestellt. Das Reaktionsgemisch wurde auf 20 bis 25 °C gekühlt, der Feststoff über eine Saugnutsche abfiltriert und zehnfach mit 150 ml Wasser gewaschen. Nach dem Trocknen im Vakuum bei 100 °C betrug die Ausbeute 321,0 g (87,8 % der Theorie). Das Produkt war laut HPLC- und NMR-Messung frei von Decansäure und nicht umgesetzter para-Hydroxybenzoesäure. Die Reinheit des Produkts ist > 99,9 Gew.-%.

### Beispiel 10: Synthese von para-Decanoyloxy-benzoesäure (DOBA)

165,7 g (1,2 mol) para-Hydroxybenzoesäure wurden zunächst in 400 ml Wasser und 600 ml Isopropanol gelöst und bei 10 bis 15 °C mit 276,1 g KOH-Lösung (50 Gew.-%ige wässrige Lösung, 2,46 mol) versetzt. Der resultierende pH-Wert war 14. Zu dieser Lösung wurden dann innerhalb von zwei Stunden bei 10 bis 15 °C 228,8 g (1,2 mol) Decansäurechlorid zudosiert und 15 Minuten bei 10 bis 15 °C nachgerührt. Am Ende des Nachrührens betrug der pH-Wert des Reaktionsansatzes 9,5. Anschließend wurde das Reaktionsgemisch bei 10 bis 15 °C mit 30 g HCl-Lösung (32 Gew.-%ige wässrige Lösung) versetzt, die komplette Lösung auf 55 bis 60 °C erhitzt und dann mit 120 g HCl-Lösung (32 Gew.-%ige wässrige Lösung) auf pH 1,5 bis 3 gestellt. Das Reaktionsgemisch wurde auf 20 bis 25 °C gekühlt, der Feststoff über eine Saugnutsche abfiltriert und zehnfach mit 150 ml Wasser gewaschen. Nach dem Trocknen im Vakuum bei 100 °C betrug die Ausbeute 314,4 g (89,6 % der Theorie). Das Produkt war laut HPLC- und NMR-Messung frei von Decansäure und nicht umgesetzter para-Hydroxybenzoesäure. Die Reinheit des Produkts ist > 99,9 Gew.-%.

### Beispiel 11: Synthese von para-Decanoyloxy-benzoesäure (DOBA)

158,8 g (1,15 mol) para-Hydroxybenzoesäure wurden zunächst in 400 ml Wasser und 600 ml Isopropanol gelöst und bei 10 bis 15 °C mit 264,8 g KOH-Lösung (50 Gew.-%ige wässrige Lösung, 2,36 mol) versetzt. Der resultierende pH-Wert war 14. Zu dieser Lösung wurden dann innerhalb von zwei Stunden bei 10 bis 15 °C 219,4 g (1,15 mol) Decansäurechlorid zudosiert und 15 Minuten bei 10 bis 15 °C nachgerührt. Am Ende des Nachrührens betrug der pH-Wert des Reaktionsansatzes 9,1. Anschließend wurde das Reaktionsgemisch bei 10 bis 15 °C mit 28,8 g HCl-Lösung (32 Gew.-%ige wässrige Lösung) versetzt, die komplette Lösung auf 55 bis 60 °C erhitzt und dann mit 115 g HCl-Lösung (32 Gew.-%ige wässrige Lösung) auf pH 1,5 bis 3 gestellt. Das Reaktionsgemisch wurde auf 20 bis 25 °C gekühlt, der Feststoff über eine Saugnutsche abfiltriert und zehnfach mit 150 ml Wasser gewaschen. Nach dem Trocknen im Vakuum bei 100 °C betrug die Ausbeute 296,1 g (88,1 % der Theorie). Das Produkt war laut HPLC- und NMR-Messung frei von Decansäure und nicht umgesetzter para-Hydroxybenzoesäure. Die Reinheit des Produkts ist > 99,9 Gew.-%.

### Beispiel 12: Synthese von para-Decanoyloxy-benzoesäure (DOBA)

138,1 g (1 mol) para-Hydroxybenzoesäure wurden zunächst in 400 ml Wasser und 600 ml Isopropanol gelöst und bei 0 bis 5 °C mit 235,7 g KOH-Lösung (50 Gew.-%ige wässrige Lösung, 2,1 mol) versetzt. Der resultierende pH-Wert war 14. Zu dieser Lösung wurden dann innerhalb von 15 Minuten bei 5 bis 25°C ohne Gegenkühlung 190,7 g (1 mol) Decansäurechlorid zudosiert und 15 Minuten nachgerührt. Am Ende des Nachrührens betrug der pH-Wert des Reaktionsansatzes 9,7. Anschließend wurde das Reaktionsgemisch bei 25 °C mit 25 g HCl-Lösung (32 Gew.-%ige wässrige Lösung) versetzt, die komplette Lösung auf 55 bis 60 °C erhitzt und dann mit 100 g HCl-Lösung (32 Gew.-%ige wässrige Lösung) auf pH 1,5 bis 3 gestellt. Das Reaktionsgemisch wurde auf 20 bis 25 °C gekühlt, der Feststoff über eine Saugnutsche abfiltriert und zehnfach mit 150 ml Wasser gewaschen. Nach dem Trocknen im Vakuum bei 100 °C betrug die Ausbeute 252,8 g (86,5 % der Theorie). Das Produkt war laut HPLC- und NMR-Messung frei von Decansäure und nicht umgesetzter para-Hydroxybenzoesäure. Die Reinheit des Produkts ist > 99,9 Gew.-%.

### Beispiel 13: Synthese von para-Decanoyloxy-benzoesäure (DOBA)

798,3 g (5,78 mol) para-Hydroxybenzoesäure wurden zunächst in 1980 ml Wasser und 2970 ml Isopropanol gelöst und bei 10 bis 15 °C mit 1329,7 g KOH-Lösung (50 Gew.-%ige wässrige Lösung, 11,84 mol) versetzt. Der resultierende pH-Wert war 14. Zu dieser Lösung wurden dann innerhalb von 100 Minuten bei 10 bis 15 °C 1106,8 g (5,78 mol) Decansäurechlorid (99,6 Gew.-% ige Lösung) zudosiert und 15 Minuten bei 10 bis 15 °C nachgerührt. Anschließend wurde das Reaktionsgemisch bei 10 bis 15 °C mit 149,1 g HCl-Lösung (31 Gew.-%ige wässrige Lösung) versetzt, die komplette Lösung auf 65 bis 70 °C erhitzt und dann mit 596,5 g HCl-Lösung (31 Gew.-%ige wässrige Lösung) auf pH 1,5 bis 3 gestellt. Das Reaktionsgemisch wurde auf 20 bis 25 °C gekühlt, der Feststoff über eine Saugnutsche abfiltriert und zehnfach mit 750 ml Wasser gewaschen. Nach dem Trocknen im Vakuum bei 100 °C betrug die Ausbeute 1487 g (88 % der Theorie). Das Produkt war laut HPLC- und NMR-Messung frei von Decansäure und nicht umgesetzter para-Hydroxybenzoesäure. Die Reinheit des Produkts ist > 99,9 Gew.-%.

## Patentansprüche

1. Verfahren zur Herstellung von Acyloxybenzoesäuren der Formel (I) 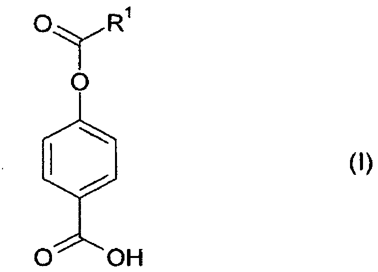 worin
R¹ eine lineare oder verzweigte, gesättigte Alkylgruppe mit 6 bis 30 Kohlenstoffatomen, eine lineare oder verzweigte, ein- oder mehrfach ungesättigte Alkenylgruppe mit 6 bis 30 Kohlenstoffatomen oder eine Arylgruppe mit 6 bis 30 Kohlenstoffatomen ist,
**dadurch gekennzeichnet, dass**
a) para-Hydroxybenzoesäure in einem Lösungsmittelgemisch enthaltend Wasser und ein organisches Lösungsmittel, wobei das eingesetzte organische Lösungsmittel iso-Propanol ist, mit einem Alkalihydroxid in einem molaren Verhältnis von Alkalihydroxid : para-Hydroxybenzoesäure ≥ 1,9 : 1 versetzt wird und erst danach ein Carbonsäurehalogenid der Formel R¹COHal, worin R¹ die oben angegebene Bedeutung besitzt und Hal ein Halogenid ist, bei einer Temperatur ≤ 25 °C zugegeben und zur Umsetzung gebracht wird,
b) das Reaktionsgemisch nach Schritt a) bei einer Temperatur ≤ 25 °C dann auf pH 6 bis 8,5 durch Zugabe von Säure eingestellt wird, wenn der pH-Wert am Ende von Schritt a) nicht bereits in diesem Bereich liegt,
c) das Reaktionsgemisch nach Schritt b), sofern dieser stattfindet und ansonsten nach Schritt a) auf eine Temperatur von 35 bis 80 °C erwärmt und danach durch Zugabe von Säure auf pH 1 bis 4 eingestellt wird, und
e) die gesamte Menge an Alkalihydroxid in Schritt a) vor der Zugabe des Carbonsäurehalogenids in den Reaktionsansatz gegeben und ansonsten in dem Verfahren keine weitere Base zugegeben wird.

2. Verfahren nach Anspruch 1, **dadurch gekennzeichnet, dass** nach Schritt c) das Reaktionsgemisch auf eine Temperatur < 35 °C abgekühlt wird (Schritt d)).

3. Verfahren nach Anspruch 1 oder 2, **dadurch gekennzeichnet, dass** das in Schritt a) eingesetzte Alkalihydroxid KOH oder NaOH ist.

4. Verfahren nach Anspruch 3, **dadurch gekennzeichnet, dass** das in Schritt a) eingesetzte Alkalihydroxid KOH ist.

5. Verfahren nach einem oder mehreren der Ansprüche 1 bis 4, **dadurch gekennzeichnet, dass** die in Schritt b) und in Schritt c) eingesetzte Säure einen pKa-Wert kleiner oder gleich 4,0 besitzt.

6. Verfahren nach einem oder mehreren der Ansprüche 1 bis 5, **dadurch gekennzeichnet, dass** die in Schritt b) und in Schritt c) eingesetzte Säure H₂SO₄ oder HCl ist.

7. Verfahren nach einem oder mehreren der Ansprüche 1 bis 6, **dadurch gekennzeichnet, dass** das Gewichtsverhältnis von Wasser zu dem einen oder den mehreren organischen Lösungsmitteln in Schritt a) von 5 : 1 bis 1 : 5 ist.

8. Verfahren nach einem oder mehreren der Ansprüche 1 bis 7, **dadurch gekennzeichnet, dass** das Gewichtsverhältnis von Wasser zu para-Hydroxybenzoesäure in Schritt a) von 2 : 1 bis 10 : 1 ist.

## Claims

1. A process for preparing acyloxybenzoic acids of the formula (I) 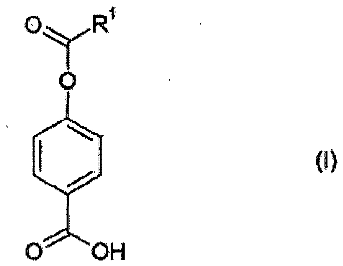 in which
R¹ is a linear or branched, saturated alkyl group having 6 to 30 carbon atoms, a linear or branched, singly or multiply unsaturated alkenyl group having 6 to 30 carbon atoms, or an aryl group having 6 to 30 carbon atoms,
which comprises
a) admixing para-hydroxybenzoic acid, in a solvent mixture comprising water and an organic solvent, the organic solvent used being isopropanol, with an alkali metal hydroxide, in an alkali metal hydroxide : para-hydroxybenzoic acid molar ratio ≥ 1.9:1, and only thereafter adding a carboxylic halide of formula R¹COHal, in which R¹ possesses the definition indicated above and Hal is a halide, at a temperature ≤ 25°C, and carrying out reaction,
b) adjusting the reaction mixture after step a), at a temperature ≤ 25°C, to a pH of 6 to 8.5 by addition of acid, if the pH at the end of step a) is not already within this range,
c) after step b), where that step takes place, and otherwise after step a), heating the reaction mixture to a temperature of 35 to 80°C and thereafter adjusting it to a pH of 1 to 4 by addition of acid, and
e) adding the entire amount of alkali metal hydroxide in step a) before the addition of the carboxylic halide to the reaction mixture, and otherwise adding no further base in the process.

2. The process as claimed in claim 1, wherein after step c) the reaction mixture is cooled to a temperature < 35°C (step d)).

3. The process as claimed in claim 1 or 2, wherein the alkali metal hydroxide used in step a) is KOH or NaOH.

4. The process as claimed in claim 3, wherein the alkali metal hydroxide used in step a) is KOH.

5. The process as claimed in one or more of claims 1 to 4, wherein the acid used in step b) and in step c) possesses a pKa value of less than or equal to 4.0.

6. The process as claimed in one or more of claims 1 to 5, wherein the acid used in step b) and in step c) is H₂SO₄ or HCl.

7. The process as claimed in one or more of claims 1 to 6, wherein the weight ratio of water to the one or more organic solvents in step a) is from 5:1 to 1:5.

8. The process as claimed in one or more of claims 1 to 7, wherein the weight ratio of water to para-hydroxybenzoic acid in step a) is from 2:1 to 10:1.

## Revendications

1. Procédé de préparation d'acides acyloxybenzoïques de formule (I) 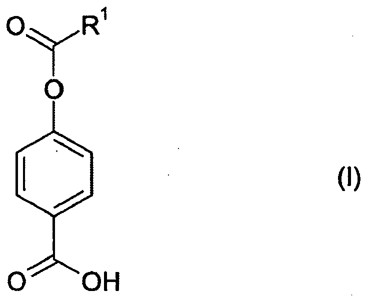 dans laquelle
R¹ est un groupe alkyle linéaire ou ramifié saturé, comprenant de 6 à 30 atomes de carbone, un groupe alcényle linéaire ou ramifié mono- ou polyinsaturé, comprenant de 6 à 30 atomes de carbone, ou un groupe aryle comprenant de 6 à 30 atomes de carbone,
**caractérisé en ce que**
a) de l'acide para-hydroxybenzoïque est mélangé dans un mélange de solvants contenant de l'eau et un solvant organique, le solvant organique utilisé étant de l'isopropanol, avec un hydroxyde alcalin dans un rapport molaire hydroxyde alcalin/acide parahydroxybenzoïque ≥ 1,9:1 et seulement après un halogénure d'acide carboxylique de formule R¹COHal, dans laquelle R¹ possède la signification indiquée ci-dessus et Hal est un halogénure, est ajouté et mis à réagir à une température ≤ 25 °C,
b) le mélange de réaction selon l'étape a) à une température ≤ 25 °C est ensuite ajusté à un pH de 6 à 8,5 par ajout d'acide si le pH ne se situe pas déjà dans cette plage à la fin de l'étape a),
c) le mélange de réaction selon l'étape b), si celle-ci a lieu, sinon selon l'étape a), est chauffé à une température de 35 à 80 °C puis ajusté à un pH de 1 à 4 par ajout d'acide, et
e) la quantité totale d'hydroxyde alcalin à l'étape a) est ajoutée au mélange de réaction avant l'ajout de l'halogénure d'acide carboxylique et à part cela aucune autre base n'est ajoutée dans le procédé.

2. Procédé selon la revendication 1, **caractérisé en ce qu'**après l'étape c), le mélange de réaction est refroidi à une température < 35 °C (étape d)).

3. Procédé selon la revendication 1 ou 2, **caractérisé en ce que** l'hydroxyde alcalin utilisé à l'étape a) est KOH ou NaOH.

4. Procédé selon la revendication 3, **caractérisé en ce que** l'hydroxyde alcalin utilisé à l'étape a) est KOH.

5. Procédé selon l'une ou plusieurs des revendications 1 à 4, **caractérisé en ce que** l'acide utilisé à l'étape b) et à l'étape c) possède un pKa inférieur ou égal à 4,0.

6. Procédé selon l'une ou plusieurs des revendications 1 à 5, **caractérisé en ce que** l'acide utilisé à l'étape b) et à l'étape c) est H₂SO₄ ou HCl.

7. Procédé selon l'une ou plusieurs des revendications 1 à 6, **caractérisé en ce que** le rapport en poids entre l'eau et le ou les solvant(s) organique(s) à l'étape a) va de 5:1 à 1:5.

8. Procédé selon l'une ou plusieurs des revendications 1 à 7, **caractérisé en ce que** le rapport en poids entre l'eau et l'acide para-hydroxybenzoïque à l'étape a) va de 2:1 à 10:1.
